# EUROPEAN PATENT SPECIFICATION

(11) **EP 4 724 829 B1**
(45) Date of publication and mention of the grant of the patent: **11.11.2026**
(21) Application number: 24731492.5
(22) Date of filing: 31.05.2024
(51) Int. Cl.: G01R 33/54, A61B 5/055

(54) **INTERFACE FOR A MEDICAL IMAGING SYSTEM**
SCHNITTSTELLE FÜR EIN MEDIZINISCHES BILDGEBUNGSSYSTEM
INTERFACE POUR UN SYSTÈME D'IMAGERIE MÉDICALE

(30) Priority: 07.06.2023 EP 23178018
(43) Date of publication of application: 15.04.2026
(73) Proprietor: Koninklijke Philips N.V., 5656 AE Eindhoven (NL)
(72) Inventor: LEUSSLER, Christoph, Günther, 5656 AG Eindhoven (NL); VERNICKEL, Peter, 5656 AG Eindhoven (NL); FINDEKLEE, Christian, 5656 AG Eindhoven (NL)
(74) Representative: Philips Intellectual Property & Standards
(86) International application number: PCT/EP2024/065023
(87) International publication number: WO 2024/251619

(56) References cited:
- DE-A1- 102015 202 795
- US-A- 5 355 885
- US-A- 5 551 430
- US-A1- 2013 154 647
- US-A1- 2022 151 494
- US-B1- 10 290 371
- US-B1- 6 356 780
- US-B2- 10 251 531
- US-B2- 9 545 497

## Description

### FIELD OF THE INVENTION

The invention relates to the field of medical imaging. In particular, the present invention relates to the field of an interface for a medical imaging system.

### BACKGROUND OF THE INVENTION

In a magnetic resonance imaging system, an interface is formed with which the signals received with the radio frequency coil can be forwarded. The radio frequency coil is part of every magnetic resonance imaging system. The radio frequency coil is used to receive the magnetic resonance signal, which is a radio frequency signal. In magnetic resonance imaging the system integrated body coil generates the excitation magnetic field for the spin system. The precession of the net magnetization induces a current in the radio frequency coil via electromagnetic induction. The radio frequency coil generally comprises a conducting wire with an inductance and a coil resistance. Today's radio frequency coils are stand-alone devices connected by a cable to the interface located at the end of the patient bed. The patient bed carries a patient and is steerable in all three directions of space so that the patient can be moved in and out of the opening of the magnetic resonance imaging system, the bore. The coil must be connected to the interface and the interface must also be connected to the patient table.

The prerequisite for improved image quality is that radio frequency coils can be placed and combined as desired. At the same time, there may be a need to switch between different types of radio frequency coils, such as analog radio frequency coils and digital radio frequency coils. The combination of radio frequency coils means that different types of radio frequency coils of one type must be combinable for different entities, for example to enable the acquisition of a whole-body image of the patient. For this purpose, common magnetic resonance imaging systems are connected to several additional components and/or systems that provide additional signals and/or information via analog and/or digital interfaces. The compatibility of the interfaces and the compatibility with the protocols is not always guaranteed.

A second source of uncertainty and also a risk is the use of spare parts or devices that are not suitable for use with the magnetic resonance imaging system and pose a risk to the patient and the functionality of the magnetic resonance imaging system. Also, the availability of additional functions or fewer functions could lead to limited compatibility and consequently to a reduction in functionality.

The publication 'Scalable Multichannel MRI Data Acquisition System' by Bodurka J, et al. in Magnetic Resonance in Medicine, 51:165-171, 2004; describes a scalable multichannel digital magnetic resonance imaging receiver system designed to achieve high bandwidth echo-planar imaging acquisitions. The modular system design allows for easy extension to an arbitrary number of channels.

WO 2006/103591 A1 discloses a magnetic resonance imaging system, a method for operating the magnetic resonance imaging system and a computer program for operating the magnetic resonance imaging system. The system comprises: an examination zone arranged to receive a body for examination; magnetic field generating means for generating a magnetic field in the examination zone; a receiving unit located in the examination zone or in the vicinity of the examination zone; an interface unit located in the examination zone or in the vicinity of the examination zone, and arranged separately from the receiving unit; and a signal processing unit disposed at a location remote from the receiving unit and the interface unit; wherein the receiving unit comprising a receiver) adapted to receive a spin resonance signal generated in the examination zone, and a transmitter adapted to transmit the spin resonance signal to the interface unit; and wherein the interface unit comprises a receiver for receiving the spin resonance signals, an analog to digital converter adapted to generate a digital signal in response to the received spin resonance signal, and a transmitter for transmitting the digitized signal to the signal processing unit. Interfaces often have the disadvantage that only radio frequency coils of a certain type can be connected. At the same time, there is no way to ensure that only radio frequency coils compatible with the magnetic resonance imaging system are used. Other peripheral devices, such as contrast medium pumps or similar, must be integrated with additional components, which also disadvantage the coordination of the peripheral devices together with the imaging system.

US 6 356 780 B1 and US 9 545 497 B2 disclose interfaces for a magnetic resonance system, the interfaces containing a host module for providing data connections for a multitude of peripheral devices.

### SUMMARY OF THE INVENTION

It is an object of the invention to provide an interface with improved compatibility.

According to the invention, this object is addressed by the subject matter of the independent claims. Preferred embodiments of the invention are described in the dependent claims.

Therefore, according to the invention, an interface for a magnetic resonance imaging system is provided, with a host module, wherein the host module is adapted for providing a multitude of data connections for a multitude of peripheral devices of different types, the host module is adapted for identifying compatible peripheral devices as well as incompatible peripheral devices, wherein the compatible peripheral devices can be employed with the host module and incompatible peripheral devices cannot be employed with the host module, the host module comprises a further data connection for transmitting data from the host module to the magnetic resonance imaging system and receiving data from the magnetic resonance imaging system with the host module, the host module is adapted for casting an interlock in the magnetic resonance imaging system when the incompatible peripheral device is identified, and the host module is adapted for identifying the type of compatible peripheral devices.

Although the invention is disclosed in relation to magnetic resonance imaging system, it should be understood that it is applicable for any medical imaging system whether is used for diagnostic purposes or in conjunction with treatment.

The host module is adapted to establish a bidirectional connection between the magnetic resonance imaging system and the peripheral devices. This allows data to be received from the peripheral devices. In addition, data can be sent to these peripheral devices so that peripheral devices which are compatible with the host module as well as their type can be identified. This generally eliminates a source of uncertainty in magnetic resonance imaging by ensuring that only compatible peripheral devices are connected to the interface. Compatibility of peripheral devices can be verified, for example, via electronic certificates included with the respective peripheral devices. When incompatible peripheral devices are connected to the interface, the interface detects them and preferably casts an interlock in the magnetic resonance imaging system.

For this purpose, it may be provided that the interlock has different severity levels. For example, the peripheral device connected to the interface may be incompatible with the magnetic field of the magnetic resonance imaging system and the respective peripheral device may pose a hazard to personnel as well as the patient. In such a case, the interlock would inhibit image acquisition. If the peripheral device is a peripheral device that exclusively affects the image quality as such, the interlock may consist in issuing a warning. The host module can be used to provide any number of the multitude of data connections for the corresponding number of peripheral devices of the multitude of peripheral devices. It is not necessary that each data connection is always provided to a peripheral device. Rather, the interface is designed to be scalable so that it can be adapted to the number of data connections provided with the corresponding number of peripheral devices.

In principle, it is possible to connect the peripheral devices to the interface in various ways. According to the invention, however, the interface further comprises a multitude of submodules of different types, wherein the submodules are interconnected between the host module and the type corresponding peripheral device, wherein the submodules have a peripheral device connector and a host module connector, the peripheral device connector of the submodule corresponding to the type of the peripheral device has the type of the submodule and the peripheral device accordingly and connects the peripheral device to the submodule for transmitting data, and the host module connectors are identical across types of submodules such that each submodule is connectable to each of the data connections provided with the host module for transmitting data, such that the host module is connected to the peripheral devices with the submodules for transmitting data from the host module to the peripheral devices and from the peripheral devices to the host module.

This makes it possible to swap the submodules with the host module connectors at the data connections. It is not necessary that a particular submodule has a host module connector that is typespecific, as is the peripheral device connector. Rather, preferably, it is possible that each submodule can be connected via the host module connection to each data connection provided by the host module, so that the interface is modular. Preferably, the peripheral device connections of the submodules are differentiated and exclusively compatible with the type of peripheral device corresponding to the submodule.

The invention provides solution to the problem of compatibility and connectivity for different peripheral devices to be compatible and safe for patient and for medical imaging systems in general, and for magnetic resonance imaging systems in particular. This is solved by individual submodules connected to the interface and the compatibility management by the integrated host module, which provides a communication channel to the individual connectable submodules (e.g., medical devices, therapy, monitoring). If compatibility and safety is in compliance, then the diagnostic system is not any more blocked from scanning or calibration processes, such as in a nonlimiting example where the interface is connected to the patient bed.

In general, the host module can be formed in different ways. According to a preferred embodiment of the invention, however, the host module is configured for controlling the peripheral devices. If the peripheral devices are designed as radio frequency coils, for example, the host module can be employed to coordinate the acquisition with the respective radio frequency coil. Similarly, if one of the peripheral devices is a contrast medium pump, the host module can control the delivery of the contrast medium.

According to a preferred embodiment of the invention, the host module is adapted for controlling a preselection of an imaging sequence based on the type of the compatible peripheral devices connected to the host module, wherein the preselection of the imaging sequence is the selection of imaging-specific parameters, wherein with the imaging sequence images are acquired with the magnetic resonance imaging system. For example, when the radio frequency coil for imaging the head, e.g. a birdcage coil, is connected to the host module, the host module is used to preselect the imaging sequence based on parameters such as field of view or similar.

It is possible to configure the connection between the host module and peripheral devices in different ways. According to a preferred embodiment of the invention, however, electrical power is supplied to the identified peripheral devices via the host module by employing the connection. For this purpose, coaxial cables can be provided to supply electrical power to the peripheral devices connected to the host module. This leads to a reduction in the number of cables in the magnetic resonance imaging system.

In principle, it is also possible for the connection between the host module and the magnetic resonance imaging system to be formed in different ways, e.g. by a wire connection or optical connection. According to an embodiment of the invention, however, the host module is connected wirelessly to the magnetic resonance imaging system.

According to a further embodiment of the invention, it is preferred that the host module connectors of the submodules and the host module are adapted for transmitting data wirelessly. The wireless connection is generally used to reduce the amount of cables. On the other hand, wired connections can be used. Such wired connections may be more advantageous, especially for the transmission of data obtained with radio frequency coils, since they are less susceptible to interference in the magnetic resonance imaging system. Optical signal transmission can be used for this purpose. In this case, the data is transmitted through an optical fiber.

Likewise, in another preferred embodiment according to the invention, it may be provided that the peripheral device connectors of the submodules and the peripheral devices are adapted for transmitting the data wirelessly or wired.

In another preferred embodiment according to the invention, the host module has an optical or acoustic feedback sensor to signal to an operator if the submodule and the host module interact with each other or when the setup is ready for use. It may take some time to load up a software driver or to check interoperability and patient safety.

According to preferred embodiment of the invention, the host module is adapted for enabling the identified peripheral devices to interact with each other via the host module. This enables cardiac gating image acquisition, for example. One of the peripheral devices is an electrocardiography device, which coordinates the image acquisition with the host module and the radio frequency coil, which is also connected, taking into account the data acquired with the electrocardiography device. The same can also be provided for respiratory gating, in that an abdominal belt as a peripheral device measures the expansion and contraction of a patient's chest and, together with the host module, coordinates the image acquisition with the radio frequency coil, which is also connected, taking into account the data from the abdominal belt. For this purpose, it is particularly advantageous that this control can be carried out directly by the host module, which enables a faster overall response time and simpler handling of these acquisition techniques.

According to another preferred embodiment of the invention, the host module is configured for extending types, such that the host module is compatible to an undefined-type peripheral device with a corresponding undefined-type submodule, wherein the undefined-type peripheral device and the undefined-type submodule cannot be identified by the host module and require configuration of the host module. Configuration of the host module in this case means updating the host module. This makes it possible to flexibly extend the compatibility of the host module to other peripheral devices and to ensure the compatibility of the host module with newer, more recent peripheral devices, so that the interface is not limited to the type of peripheral devices that existed at the time the interface was developed.

In general, different types of submodules and peripheral devices can be connected to the host module. According to a preferred embodiment of the invention, however, the interface is connectable with first-type submodules and second-type submodules. Preferably, the first-type submodules are connectable to first-type peripheral devices which are analog radio frequency coils and the second-type submodules are connectable to second-type peripheral devices which are digital radio frequency coils. This refers to all radio frequency coils, such as birdcage coils or body coils or other radio frequency coils of one type. The type itself only defines the differences between analog and digital radio frequency coils. The host module is designed to recognize the types of peripheral devices. Based on the detected types of the peripheral devices, a preselection of the imaging sequence can be performed using the radio frequency coils connected to the host module. Different radio frequency coils are provided in the different types. That is, birdcage coils or body coils or other radio frequency coils preferred for individual examination entities may be provided. Likewise, radio frequency coils can be provided that are designed for different magnetic fields in the different types.

According to a preferred embodiment of the invention, the interface further comprises third-type submodules and a fourth-type submodules, wherein the third-type submodules are connectable to third-type peripheral devices that are sensors for measuring vital parameters, and the fourth type submodules are connectable to fourth type peripheral devices that are software-defined radios. The sensors for measuring vital parameters can be the electrocardiogram device mentioned above and the abdominal belt. Further sensors can be provided, which measure vital parameters like a respiratory rate, a temperature and/or a pulse of the patient. The software-defined radio platform is typically used for signal processing and transmitting and receiving signals. A basic software-defined radio architecture consists of a radio front end, an analog-to-digital converter, a digital-to-analog converter and a digital signal processor. Software-defined radio-based systems can operate reliably and accurately over a large bandwidth and frequency spectrum, making them a good platform to host ultra-wideband wireless technology, which is becoming increasingly popular in hospital environments. A medical imaging system is considered ultra-wideband when the range of its operating frequency is greater than 500 MHz. The higher frequencies resolve the finer structures, while the lower frequencies resolve larger structures and provide contrast to the reconstructed image.

According to a preferred embodiment of the invention, the interface additionally comprises further types of submodules, wherein the further-type submodules are connectable to corresponding peripheral devices that are, for example, data carriers, contrast agent pumps, respirators, perfusers, a connection unit, wherein the connection unit is connectable to a wearable health monitor, a patient communication system, an entertainment system and/or service tooling. The configured interface should be safe for the patient and be electromagnetically compatible. Therefore, according to a preferred embodiment of the invention, new submodules or new combinations of submodules with the host device are checked for patient safety and/or image quality. Thus, in case of concern, a dedicated compatibility check may be initiated by an operator or a remote control service to proof and certify patient safety.

In a further preferred embodiment of the invention, the interface comprises a remote control, whereby the remote control is connected to the host module via the further data connection and is adapted for controlling the host module and to retrieve data from the peripheral devices. In principle, the remote control can be designed in different ways. With the remote control, the patient's vital parameters can be viewed if the third-type peripheral devices are connected to the host module in an interactive mode during image acquisition. According to a preferred embodiment of the invention, however, the remote control comprises a quick response code scanner. This allows patients wearing a corresponding wristband with a quick response code to be identified.

Preferably, the interface further comprises a fluidic connection with which a connection is established analogously to the further data connection and the data connection between the magnetic resonance tomography system and the peripheral device, wherein a fluid is provided to the peripheral device via the host module with the fluidic connection. This enables the supply of oxygen to a respirator that is connected to the interface as a further-type peripheral device.

Further, according to the invention a magnetic resonance imaging system with an interface according to the above is provided, with a magnetic resonance sequencer and at least one of an illumination, a ventilation and a communication unit, wherein the magnetic resonance sequencer is adapted for executing an imaging sequence with the magnetic resonance imaging system upon the preselection of the imaging sequence determined with the host module, and the host module is adapted for controlling environmental parameters, wherein the environmental parameters are at least one of the illumination, the ventilation and an acoustic adaptation of a communication realizable with the communication unit based on a noise gradient of the magnetic resonance imaging system. The magnetic resonance sequencer is used to execute the imaging sequence with the magnetic resonance imaging system. The magnetic resonance sequencer is designed as a component of the magnetic resonance imaging system and can control the radio frequency pulse and the gradient magnets. It also processes the data received with the radio frequency coils. The magnetic resonance sequencer has an input facility that can be used to control the image sequence and the environmental parameters mentioned above. Since the host module can control the environmental parameters, there is no need for additional input into the magnetic resonance sequencer. Rather, the host module can be used to transmit a preselected imaging sequence to the magnetic resonance sequencer that requires confirmation or modification by personnel. The host module can then control the environmental parameters based on the imaging sequence executed by the magnetic resonance sequencer.

In principle, it is possible to connect different components of the magnetic resonance imaging system to the interface. According to a preferred embodiment of the invention, however, the magnetic resonance imaging system comprises a patient bed, wherein the interface is integrally formed with the patient bed, and the patient bed is a submodule and connectable to peripheral devices of corresponding types.

In general, it is possible that the patient bed can be connected to different types of peripheral devices. According to a preferred embodiment of the invention, however, the patient bed is connectable to the first-type peripheral devices and the second-type peripheral devices.

By making the interface integral with the patient bed, the number of devices and cables in the magnetic resonance imaging system is reduced. The peripheral devices can be connected with the peripheral device connectors formed in the patient bed. The patient bed is connected to the host module via the host module connectors. First-type peripheral devices and second-type peripheral devices can be plugged directly into the patient bed, reducing the distance for data transmission of data received with the radio frequency coils. This makes the data less susceptible to interference from the radiated radio frequency fields of the magnetic resonance imaging system.

In principle, it is possible to design the patient bed in different ways. According to a preferred embodiment of the invention, however, the magnetic resonance imaging system further comprises a controller, the controller being interconnected between the patient bed and the host module, wherein the patient bed is a peripheral device and the controller corresponds to the corresponding submodule, so that the patient bed is controllable with the host module.

With the controller, it is possible to use the patient bed not only as a submodule for the peripheral devices, but rather as a peripheral device itself. This allows the patient bed to be moved with the interface to desired positions within the bore during the imaging sequence. According to a further embodiment of the invention, the patient bed comprises an interlock with which the connections to the peripheral devices can be blocked. In this way, a mechanical interlock can be cast with the patient bed so that the peripheral devices cannot be connected to the interface in the first place.

The invention further relates to a method of operating a magnetic resonance imaging system, comprising the following method steps: connecting the peripheral devices to the interface, identifying compatible peripheral devices as well as incompatible peripheral devices, casting an interlock in the magnetic resonance imaging system when an incompatible peripheral device is identified, identifying the type of the compatible peripheral devices, preselecting of an imaging sequence with the interface upon identification of the compatible peripheral devices, establishing the further data connection from the interface to the magnetic resonance imaging system, and performing the imaging sequence with the magnetic resonance imaging system if no interlock has occurred.

Further, according to the invention, a method of operating a magnetic resonance imaging system is provided, comprising the following method steps: connecting the peripheral devices to the patient bed, identifying peripheral devices connected to the patient bed, casting the interlock in the magnetic resonance imaging system when the incompatible peripheral device is identified, identifying the type of the compatible peripheral devices, preselecting of an imaging sequence with the patient bed upon identification of compatible peripheral devices, establishing the further data connection from the patient bed to the magnetic resonance imaging system, and performing the imaging sequence with the magnetic resonance imaging system if no interlock has occurred. For this purpose, it is provided that the interface is integrally formed in the patient bed.

According to a preferred embodiment of the invention, the method of operating a magnetic resonance imaging system comprises the following further step: configuring the scalable interface so that undefined submodule types and undefined peripheral devices are compatible.

In a further preferred embodiment of the invention, the method of operating a magnetic resonance imaging system comprises the following further step: transmitting the imaging sequence to the interface, wherein during step of performing the imaging sequence with the magnetic resonance imaging system if no interlock has occurred, the following further step is executed: controlling environmental parameters with the interface.

According to a preferred embodiment of the invention, the method of operating a magnetic resonance imaging system further comprises the remote control and the following further steps: connecting the remote control to the scalable interface, and transmitting signals to the remote control with the scalable interface.

The invention further comprises a computer-readable medium in accordance with claim 14, comprising instructions thereon which, when executed by an interface in a magnetic resonance imaging system, cause the interface to carry out a method as described above.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the system, as defined in the claims, is provided in the following written description of various embodiments of the disclosure and illustrated in the accompanying drawings.

### BRIEF DESCRIPTION OF THE DRAWINGS

These and other aspects of the invention will be apparent from and elucidated with reference to the embodiments described hereinafter. Such an embodiment does not necessarily represent the full scope of the invention.
In the drawings:
Fig. 1 schematically depicts an interface according to a preferred embodiment of the invention;
Fig. 2 schematically depicts an interface according to another preferred embodiment of the invention;
Fig. 3 schematically depicts a magnetic resonance imaging system according to a preferred embodiment of the invention; and
Fig. 4 schematically depicts a method according to a preferred embodiment of the invention.

### DETAILED DESCRIPTION OF EMBODIMENTS

For the purposes of promoting and understanding the principles of the present disclosure, reference will now be made to the embodiments illustrated in the drawings, and specific language will be used to describe the same. It is nevertheless understood that no limitation to the scope of the disclosure is intended. Any alteration and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately. Features described in relation to a system, may be implemented in a computer implemented method and/or in a computer program product, in a corresponding manner.

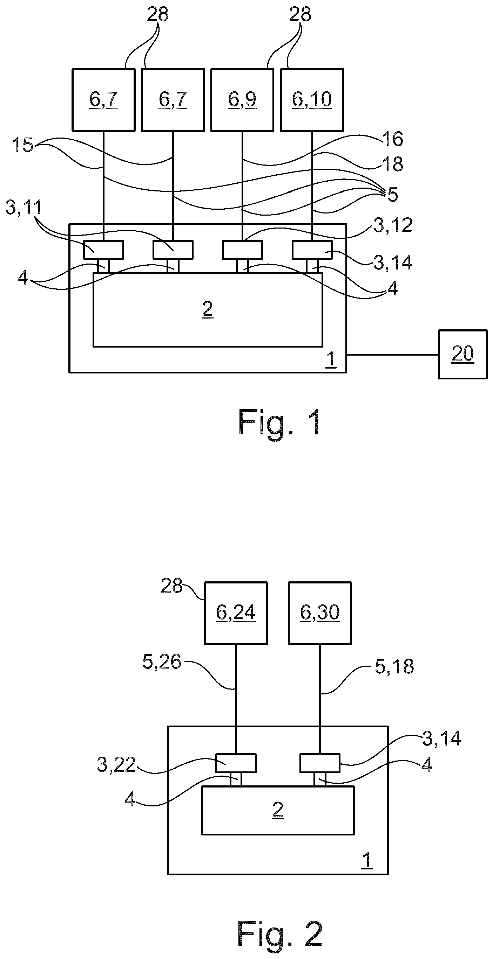

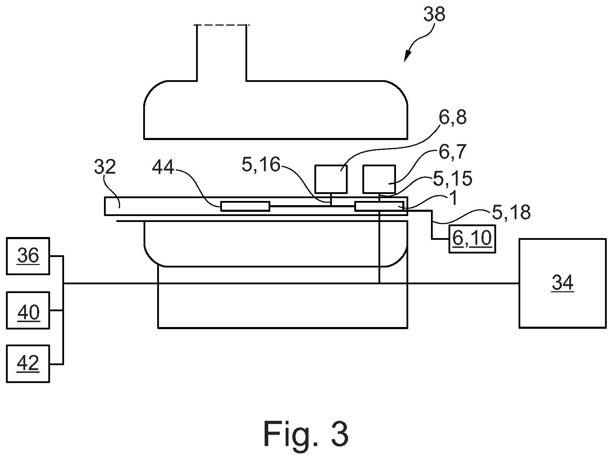

Fig. 1 schematically depicts an interface 1 according to a preferred embodiment of the invention. The interface 1 has a host module 2, which is connected via host module connectors 4 to submodules 3 for transmitting data. The submodules 3 are also connected to peripheral devices 6 via peripheral device connectors 5 in such a way that data can be transmitted. The host module 2 provides corresponding data connections for connection to the peripheral devices 6 via the submodules 3. The peripheral devices 6 connected to the host module 2 via the submodules 3 are all compatible peripheral devices 28. A remote control 20 is also connected to the host module 2 shown in Fig. 1 via a further data connection. With the further data connection, the host module 2 is connected to the magnetic resonance imaging device 38 shown in Fig. 3 for data transmission. The remote control 20 can be used to view vital parameters that can be measured with the third-type peripheral device 10 connected to the host module 2. For this purpose, the third-type peripheral device 10 is connected to a third-type submodule 14 with a third-type peripheral device connector 18 corresponding to the type. The third-type submodule 14 is connected to the host module 2 via the host module connector 4. The host module 2 in Fig. 1 is also connected to two host module connectors 4 via two first-type submodules 11 and two first-type peripheral device connectors 15, each with a first-type peripheral device 7. The host module 2 is also connected to a second-type peripheral device 8 by means of a host module connector 4 via a second-type submodule 12 and a second-type peripheral device connector 16.

Fig. 2 schematically depicts the interface 1 according to another preferred embodiment of the invention. The host module 2 is connected to an undefined-type submodule 22 via the host module connector 4. The undefined-type submodule 22 is connected to an undefined-type peripheral device 24 via an undefined-type peripheral device connector 26. The host module 2 is now configurable to identify the undefined-type submodule 22 and the undefined-type peripheral device 24, and to recognize the undefined-type peripheral device 24 as a compatible peripheral device 28. The host module 2 is still connected to the third-type submodule 14 via the host module connector 4. The third-type submodule 14 is connected to an incompatible peripheral device 30 via the third-type peripheral device connector 18, whereupon an interlock is cast in the magnetic resonance imaging system.

Fig. 3 schematically depicts the magnetic resonance imaging system 38 according to a preferred embodiment of the invention. The magnetic resonance imaging system 38 includes a patient bed 32 and the interface 1, which may be integrally formed in the patient bed 32. In addition, the patient bed 32 has a controller 44 connected to the interface 1 such that the interface 1 can control the patient bed 32 as a peripheral device 6. Furthermore, the patient bed 32 is a submodule 3 and is connected via the first-type peripheral device connector 15, the second-type peripheral device connector 16 and the third-type peripheral device connector 18 to the first-type peripheral device 7, the second-type peripheral device 8 and the third-type peripheral device 10, respectively. Via the further data connection, the interface 1 is connected to a magnetic resonance sequencer 34 as well as an illumination 36, a ventilation 40 and a communication unit 42. The interface 1 sends a preselected imaging sequence to the magnetic resonance sequencer 34 based on the connected peripheral devices 6, and controls the environmental parameters in the form of the illumination 36, the ventilation 40 and an acoustic adaptation of a communication realizable with the communication unit 42 based on a noise gradient of the magnetic resonance imaging system 38 during the image acquisition with the magnetic resonance imaging system 38.

In any of the embodiments the individual submodules (bidirectional) may have an identical connector to the interface, so that it can be connected to any of the identical sockets. The connection of the peripheral device is based on the individual connector of the dedicated peripheral device. This can also be galvanically connected or partly wireless. The socket module may contain an internal controller (analog or digital controller, microcontroller, etc.) to provide compatibility or to adapt control signals in amplitude, phase, frequency, time) and provide compatibility.

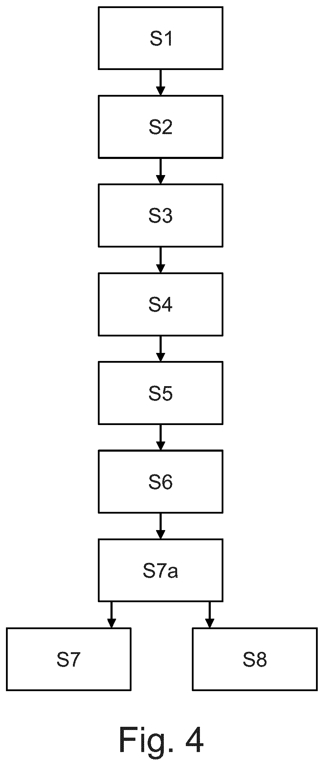

Fig. 4 schematically depicts a scheme of a method according to a preferred embodiment of the invention, wherein in a first step S1 the peripheral devices 6 are connected to the patient bed 32. Second, in a further step S2 the peripheral devices 6 connected to the patient bed 32 are identified. In a third step S3, an interlock is cast in the magnetic resonance imaging system 38, when the incompatible peripheral device 30 is identified. Fourth, in a further step S4 the types of the compatible peripheral devices 28 are identified. In a fifth step S5, an imaging sequence is preselected with the patient bed 32 upon identification of the compatible peripheral devices 28. Sixth, the further data connection from the patient bed 32 to the magnetic resonance imaging system is established. In a further step S7a, the imaging sequence is transmitted to the interface 1 in the patient bed 32. In the following step, the imaging sequence is performed with the magnetic resonance imaging system 38 if no interlock has occurred. During this step the environmental parameters are controlled with the interface 1 in the patient bed 32.

It is understood that one or more of the aforementioned embodiments of the invention may be combined as long as the combined embodiments fall within the scope of the claims and are not mutually exclusive.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope. Further, for the sake of clearness, not all elements in the drawings may have been supplied with reference signs.

### REFERENCE SYMBOL LIST

| | |
|---|---|
| Interface | 1 |
| host module | 2 |
| submodule | 3 |
| host module connector | 4 |
| peripheral device connector | 5 |
| peripheral device | 6 |
| first-type peripheral device | 7 |
| second-type peripheral device | 8 |
| third-type peripheral device | 10 |
| first-type submodule | 11 |
| second-type submodule | 12 |
| third-type submodule | 14 |
| first-type peripheral device connector | 15 |
| second-type peripheral device connector | 16 |
| third-type peripheral device connector | 18 |
| remote control | 20 |
| undefined-type submodule | 22 |
| undefined-type peripheral device | 24 |
| undefined-type peripheral device connector | 26 |
| compatible peripheral device | 28 |
| incompatible peripheral device | 30 |
| patient bed | 32 |
| magnetic resonance sequencer | 34 |
| illumination | 36 |
| magnetic resonance imaging system | 38 |
| ventilation | 40 |
| communication unit | 42 |
| controller | 44 |

## Claims

1. An interface (1) for a medical imaging system (38), with a host module (2) and a multitude of submodules (3) of different types, wherein
the host module (2) is adapted for providing a multitude of data connections for a multitude of peripheral devices (6) of different types,
the submodules (3) are interconnected between the host module (2) and the type of the corresponding peripheral device (6),
the host module (2) is adapted for identifying compatible peripheral devices (28) as well as incompatible peripheral devices (30), wherein the compatible peripheral devices (30) can be employed with the host module (2) and incompatible peripheral devices (28) cannot be employed with the host module,
the host module (2) comprises a further data connection for transmitting data from the host module (2) to the medical imaging system (38) and receiving data from the medical imaging system (38) with the host module (2),
the host module (2) is adapted for casting an interlock in the medical imaging system (38) when the incompatible peripheral device (30) is identified, and
the host module (2) is adapted for identifying the type of compatible peripheral devices (28) wherein
each of the submodules (3) has a peripheral device connector (5) and a host module connector (4),
the peripheral device connector (5) of the submodule (3) corresponding to the type of the peripheral device (6) has the type of the submodule (3) and the peripheral device (6) accordingly and connects the peripheral device (6) to the submodule (3) for transmitting data, and
the host module connectors (4) are identical across types of submodules (3) such that each submodule (3) is connectable to each of the data connections provided with the host module (2) for transmitting data, such that the host module (2) is connected to the peripheral devices (6) with the submodules (3) for transmitting data from the host module to the peripheral devices (6) and from the peripheral devices (6) to the host module (2).

2. The interface (1) according to claim 1, wherein the host module (2) is configured for controlling the peripheral devices (6).

3. The interface according to claim 2, wherein the host module (2) is adapted for controlling a preselection of an imaging sequence based on the type of the compatible peripheral devices (28) connected to the host module (2), wherein
the preselection of the imaging sequence, is the selection of imaging-specific parameters, wherein with the imaging sequence images are acquired with the medical imaging system (38).

4. The interface (1) according to one of the claims 1 to 3, wherein the host module (2) is configured for extending types, such that the host module (2) is compatible to an undefined-type peripheral device (24) with a corresponding undefined-type submodule (22), wherein
the undefined-type peripheral device (24) and the undefined-type submodule (22) cannot be identified by the host module and require configuration of the host module.

5. The interface (1) according to any one of the preceding claims, with first-type submodules (11) and second-type submodules (12), wherein
the first-type submodules (11) are connectable to first-type peripheral devices (7) which are analog radio frequency coils and the second-type submodules (12) are connectable to second-type peripheral devices (8) which are digital radio frequency coils.

6. The interface (1) according to any one of the preceding claims, with a remote control (20), whereby the remote control (20) is connected to the host module (2) via the further data connection and is adapted for controlling the host module (2) and to retrieve data from the peripheral devices (6).

7. A magnetic resonance imaging system (38) with an interface (1) according to any one of the preceding claims, with a magnetic resonance sequencer (34) and at least one of an illumination (36), a ventilation (40) and a communication unit (42), wherein
the magnetic resonance sequencer (34) is adapted for executing an imaging sequence with the magnetic resonance imaging system (38) upon the preselection of the imaging sequence determined with the host module (2), and
the host module (2) is adapted for controlling environmental parameters, wherein the environmental parameters are at least one of the illumination (36), the ventilation (40) and an acoustic adaptation of a communication realizable with the communication unit (42) based on a noise gradient of the magnetic resonance imaging system (38).

8. The magnetic resonance imaging system (38) according to claim 7, with a patient bed (32), wherein
the interface (1) is integrally formed with the patient bed (32), and
the patient bed (32) is one submodule (3) and is connectable to peripheral devices (6) of corresponding types.

9. The magnetic resonance imaging system (38) according to claim 8, when dependent on claim 5, wherein the patient bed (32) is connectable to the first-type peripheral devices (7) and the second-type peripheral devices (8).

10. The magnetic resonance imaging system (38) according to claim 8 or 9 with a controller (44), the controller (44) being interconnected between the patient bed (32) and the host module (2), wherein the patient bed (32) is a peripheral device (6) and the controller (44) corresponds to the corresponding submodule (3), so that the patient bed (32) is controllable with the host module (2).

11. A method of operating a medical imaging system (38) according to any one of claims 7 to 10, comprising:
connecting (S1) the peripheral devices (6) to the interface (1),
identifying (S2) the compatible peripheral devices (28) as well as incompatible peripheral devices (30),
casting (S3) the interlock in the medical imaging system (38) when the incompatible peripheral device (30) is identified,
identifying (S4) the types of the compatible peripheral devices (28),
preselecting (S5) an imaging sequence with the interface (1) upon identification of the compatible peripheral devices (28),
establishing (S6) the further data connection from the interface (1) to the medical imaging system (38), and
performing (S7) the imaging sequence with the medical imaging system (38) if no interlock has occurred.

12. A method of operating a medical imaging system (38) according to any one of claims 7 to 10, comprising:
connecting (S1) the peripheral devices (6) to the patient bed (32),
identifying (S2) the peripheral devices (6) connected to the patient bed (32),
casting (S3) the interlock in the medical imaging system (38) when the incompatible peripheral device (30) is identified,
identifying (S4) the types of the compatible peripheral devices (28),
preselecting (S5) an imaging sequence with the patient bed (32) upon identification of the compatible peripheral devices (28),
establishing (S6) the further data connection from the patient bed (32) to the medical imaging system (38), and
performing (S7) the imaging sequence with the medical imaging system (38) if no interlock has occurred.

13. The method according to claim 11 or 12, further comprising:
transmitting (S7a) the imaging sequence to the interface (1), and wherein during step S7 the following further step is executed:
controlling (S8) environmental parameters with the interface (1).

14. A computer-readable medium comprising instructions thereon which, when executed by an interface (1) according to any one of the claims 1-6 in a medical imaging system (38), cause the interface (1) to carry out a method according to any one of claims 11 to 13.

## Patentansprüche

1. Schnittstelle (1) für ein medizinisches Bildgebungssystem (38) mit einem Hostmodul (2) und einer Vielzahl von Submodulen (3) unterschiedlicher Typen, wobei
das Hostmodul (2) zum Bereitstellen einer Vielzahl von Datenverbindungen für eine Vielzahl von Peripheriegeräten (6) unterschiedlicher Typen ausgelegt ist,
die Submodule (3) dem Hostmodul (2) und dem Typ des entsprechenden Peripheriegeräts (6) zwischengeschaltet sind,
das Hostmodul (2) zum Identifizieren kompatibler Peripheriegeräte (28) sowie inkompatibler Peripheriegeräte (30) ausgelegt ist, wobei die kompatiblen Peripheriegeräte (30) mit dem Hostmodul (2) verwendet werden können und inkompatible Peripheriegeräte (28) nicht mit dem Hostmodul verwendet werden können,
das Hostmodul (2) eine weitere Datenverbindung zum Übertragen von Daten von dem Hostmodul (2) an das medizinische Bildgebungssystem (38) und zum Empfangen von Daten von dem medizinischen Bildgebungssystem (38) mit dem Hostmodul (2) umfasst,
das Hostmodul (2) zum Auslösen einer Verriegelung in dem medizinischen Bildgebungssystem (38), wenn das inkompatible Peripheriegerät (30) identifiziert wird, ausgelegt ist und
das Hostmodul (2) zum Identifizieren des Typs kompatibler Peripheriegeräte (28) ausgelegt ist, wobei jedes der Submodule (3) einen Peripheriegeräteanschluss (5) und einen Hostmodulanschluss (4) aufweist,
der Peripheriegeräteanschluss (5) des Submoduls (3), das dem Typ des Peripheriegeräts (6) entspricht, folglich den Typ des Submoduls (3) und des Peripheriegeräts (6) aufweist und das Peripheriegerät (6) mit dem Submodul (3) zum Übertragen von Daten verbindet, und
die Hostmodulanschlüsse (4) über alle Typen von Submodulen (3) hinweg identisch sind, so dass jedes Submodul (3) mit jeder der mit dem Hostmodul (2) zum Übertragen von Daten versehenen Datenverbindungen verbindbar ist, so dass das Hostmodul (2) mit den Peripheriegeräten (6) mit den Submodulen (3) zum Übertragen von Daten von dem Hostmodul an die Peripheriegeräte (6) und von den Peripheriegeräten (6) an das Hostmodul (2) verbunden ist.

2. Schnittstelle (1) nach Anspruch 1, wobei das Hostmodul (2) zum Steuern der Peripheriegeräte (6) konfiguriert ist.

3. Schnittstelle nach Anspruch 2, wobei das Hostmodul (2) zum Steuern einer Vorauswahl einer Bildgebungssequenz basierend auf dem Typ der kompatiblen Peripheriegeräte (28), die mit dem Hostmodul (2) verbunden sind, ausgelegt ist, wobei
die Vorauswahl der Bildgebungssequenz die Auswahl bildgebungsspezifischer Parameter ist, wobei mit der Bildgebungssequenz Bilder mit dem medizinischen Bildgebungssystem (38) erfasst werden.

4. Schnittstelle (1) nach einem der Ansprüche 1 bis 3, wobei das Hostmodul (2) zum Erweitern von Typen konfiguriert ist, so dass das Hostmodul (2) mit einem Peripheriegerät (24) eines undefinierten Typs mit einem entsprechenden Submodul (22) eines undefinierten Typs kompatibel ist, wobei
das Peripheriegerät (24) eines undefinierten Typs und das Submodul (22) eines undefinierten Typs von dem Hostmodul nicht identifiziert werden können und eine Konfiguration des Hostmoduls erfordern.

5. Schnittstelle (1) nach einem der vorhergehenden Ansprüche mit Submodulen (11) eines ersten Typs und Submodulen (12) eines zweiten Typs, wobei die Submodule (11) eines ersten Typs mit Peripheriegeräten (7) eines ersten Typs, die analoge Hochfrequenzspulen sind, verbindbar sind und die Submodule (12) eines zweiten Typs mit Peripheriegeräten (8) eines zweiten Typs, die digitale Hochfrequenzspulen sind, verbindbar sind.

6. Schnittstelle (1) nach einem der vorhergehenden Ansprüche mit einer Fernbedienung (20), wobei die Fernbedienung (20) über die weitere Datenverbindung mit dem Hostmodul (2) verbunden ist und zum Steuern des Hostmoduls (2) und Abrufen von Daten aus den Peripheriegeräten (6) ausgelegt ist.

7. Magnetresonanzbildgebungssystem (38) mit einer Schnittstelle (1) nach einem der vorhergehenden Ansprüche mit einer Magnetresonanz-Sequenzsteuerung (34) und einer Beleuchtung (36) und/oder einer Belüftung (40) und/oder einer Kommunikationseinheit (42), wobei die Magnetresonanz-Sequenzsteuerung (34) zum Ausführen einer Bildgebungssequenz mit dem Magnetresonanzbildgebungssystem (38) bei der Vorauswahl der mit dem Hostmodul (2) bestimmten Bildgebungssequenz ausgelegt ist und
das Hostmodul (2) zum Steuern von Umgebungsparametern ausgelegt ist, wobei die Umgebungsparameter die Beleuchtung (36) und/oder die Belüftung (40) und/oder eine akustische Anpassung einer Kommunikation sind, die mit der Kommunikationseinheit (42) basierend auf einem Geräuschgradienten des Magnetresonanzbildgebungssystems (38) realisierbar ist.

8. Magnetresonanzbildgebungssystem (38) nach Anspruch 7 mit einer Patientenliege (32), wobei
die Schnittstelle (1) mit der Patientenliege (32) integral ausgebildet ist und
die Patientenliege (32) ein Submodul (3) ist und mit Peripheriegeräten (6) entsprechender Typen verbindbar ist.

9. Magnetresonanzbildgebungssystem (38) nach Anspruch 8 bei Abhängigkeit von Anspruch 5, wobei die Patientenliege (32) mit den Peripheriegeräten (7) eines ersten Typs und den Peripheriegeräten (8) eines zweiten Typs verbindbar ist.

10. Magnetresonanzbildgebungssystem (38) nach Anspruch 8 oder 9 mit einer Steuerung (44), wobei die Steuerung (44) der Patientenliege (32) und dem Hostmodul (2) zwischengeschaltet ist, wobei die Patientenliege (32) ein Peripheriegerät (6) ist und die Steuerung (44) dem entsprechenden Submodul (3) entspricht, so dass die Patientenliege (32) mit dem Hostmodul (2) steuerbar ist.

11. Verfahren zum Betreiben eines medizinischen Bildgebungssystems (38) nach einem der Ansprüche 7 bis 10, das Folgendes umfasst:
Verbinden (S1) der Peripheriegeräte (6) mit der Schnittstelle (1),
Identifizieren (S2) der kompatiblen Peripheriegeräte (28) sowie inkompatibler Peripheriegeräte (30), Auslösen (S3) der Verriegelung in dem medizinischen Bildgebungssystem (38), wenn das inkompatible Peripheriegerät (30) identifiziert wird,
Identifizieren (S4) der Typen der kompatiblen Peripheriegeräte (28),
Vorauswählen (S5) einer Bildgebungssequenz mit der Schnittstelle (1) bei einer Identifizierung der kompatiblen Peripheriegeräte (28),
Herstellen (S6) der weiteren Datenverbindung von der Schnittstelle (1) zu dem medizinischen Bildgebungssystem (38) und
Durchführen (S7) der Bildgebungssequenz mit dem medizinischen Bildgebungssystem (38), wenn keine Verriegelung aufgetreten ist.

12. Verfahren zum Betreiben eines medizinischen Bildgebungssystems (38) nach einem der Ansprüche 7 bis 10, das Folgendes umfasst:
Verbinden (S1) der Peripheriegeräte (6) mit der Patientenliege (32),
Identifizieren (S2) der mit der Patientenliege (32) verbundenen Peripheriegeräte (6),
Auslösen (S3) der Verriegelung in dem medizinischen Bildgebungssystem (38), wenn das inkompatible Peripheriegerät (30) identifiziert wird,
Identifizieren (S4) der Typen der kompatiblen Peripheriegeräte (28),
Vorauswählen (S5) einer Bildgebungssequenz mit der Patientenliege (32) bei einer Identifizierung der kompatiblen Peripheriegeräte (28),
Herstellen (S6) der weiteren Datenverbindung von der Patientenliege (32) zu dem medizinischen Bildgebungssystem (38) und
Durchführen (S7) der Bildgebungssequenz mit dem medizinischen Bildgebungssystem (38), wenn keine Verriegelung aufgetreten ist.

13. Verfahren nach Anspruch 11 oder 12, das ferner Folgendes umfasst:
Übertragen (S7a) der Bildgebungssequenz an die Schnittstelle (1), wobei während des Schritts S7 der folgende weitere Schritt ausgeführt wird:
Steuern (S8) von Umgebungsparametern mit der Schnittstelle (1).

14. Computerlesbares Medium, das Anweisungen darauf umfasst, die, wenn sie durch eine Schnittstelle (1) nach einem der Ansprüche 1 bis 6 in einem medizinischen Bildgebungssystem (38) ausgeführt werden, bewirken, dass die Schnittstelle (1) ein Verfahren nach einem der Ansprüche 11 bis 13 durchführt.

## Revendications

1. Interface (1) pour un système d'imagerie médicale (38), avec un module hôte (2) et une multitude de sous-modules (3) de types différents, dans laquelle le module hôte (2) est conçu pour fournir une multitude de connexions de données pour une multitude de dispositifs périphériques (6) de différents types,
les sous-modules (3) sont interconnectés entre le module hôte (2) et le type du dispositif périphérique (6) correspondant,
le module hôte (2) est conçu pour identifier des dispositifs périphériques compatibles (28) ainsi que des dispositifs périphériques incompatibles (30), les dispositifs périphériques compatibles (30) pouvant être employés avec le module hôte (2) et les dispositifs périphériques incompatibles (28) ne pouvant pas être employés avec le module hôte,
le module hôte (2) comprend une connexion de données supplémentaire permettant de transmettre des données du module hôte (2) au système d'imagerie médicale (38) et de recevoir des données du système d'imagerie médicale (38) avec le module hôte (2),
le module hôte (2) est conçu pour activer un verrouillage de sécurité dans le système d'imagerie médicale (38) lorsque le dispositif périphérique incompatible (30) est identifié, et
le module hôte (2) est conçu pour identifier le type de dispositifs périphériques compatibles (28) dans lequel chacun des sous-modules (3) présente un connecteur de dispositif périphérique (5) et un connecteur de module hôte (4),
le connecteur de dispositif périphérique (5) du sous-module (3) correspondant au type du dispositif périphérique (6) présente le type du sous-module (3) et le dispositif périphérique (6) en conséquence et connecte le dispositif périphérique (6) au sous-module (3) pour transmettre des données, et
les connecteurs de module hôte (4) sont identiques sur des types de sous-modules (3) de telle sorte que chaque sous-module (3) puisse être connecté à chacune des connexions de données fournies avec le module hôte (2) pour transmettre des données, de telle sorte que le module hôte (2) soit connecté aux dispositifs périphériques (6) avec les sous-modules (3) pour transmettre des données du module hôte aux dispositifs périphériques (6) et des dispositifs périphériques (6) au module hôte (2).

2. Interface (1) selon la revendication 1, dans laquelle le module hôte (2) est configuré pour commander les dispositifs périphériques (6).

3. Interface selon la revendication 2, dans laquelle le module hôte (2) est conçu pour commander une présélection d'une séquence d'imagerie sur la base du type des dispositifs périphériques compatibles (28) connectés au module hôte (2), dans laquelle la présélection de la séquence d'imagerie est la sélection de paramètres spécifiques à l'imagerie, les images de la séquence d'imagerie étant acquises avec le système d'imagerie médicale (38).

4. Interface (1) selon l'une des revendications 1 à 3, dans laquelle le module hôte (2) est configuré pour étendre des types, de telle sorte que le module hôte (2) soit compatible avec un dispositif périphérique de type indéfini (24) avec un sous-module de type indéfini (22) correspondant, dans laquelle
le dispositif périphérique de type indéfini (24) et le sous-module de type indéfini (22) ne peuvent pas être identifiés par le module hôte et nécessitent une configuration du module hôte.

5. Interface (1) selon l'une quelconque des revendications précédentes, avec des sous-modules de premier type (11) et des sous-modules de second type (12), dans laquelle
les sous-modules de premier type (11) peuvent être connectés à des dispositifs périphériques de premier type (7) qui sont des bobines radiofréquence analogiques et les sous-modules de second type (12) peuvent être connectés à des dispositifs périphériques de second type (8) qui sont des bobines radiofréquence numériques.

6. Interface (1) selon l'une quelconque des revendications précédentes, dotée d'une télécommande (20), la télécommande (20) étant connectée au module hôte (2) par l'intermédiaire de la connexion de données supplémentaire et est conçue pour commander le module hôte (2) et pour récupérer des données des dispositifs périphériques (6).

7. Système d'imagerie par résonance magnétique (38) doté d'une interface (1) selon l'une quelconque des revendications précédentes, doté d'un séquenceur de résonance magnétique (34) et d'un éclairage (36) et/ou d'une ventilation (40) et/ou d'une unité de communication (42), dans lequel
le séquenceur de résonance magnétique (34) est conçu pour exécuter une séquence d'imagerie avec le système d'imagerie par résonance magnétique (38) lors de la présélection de la séquence d'imagerie déterminée avec le module hôte (2), et
le module hôte (2) est conçu pour commander des paramètres environnementaux, les paramètres environnementaux étant l'éclairage (36) et/ou la ventilation (40) et/ou une adaptation acoustique d'une communication réalisable avec l'unité de communication (42) sur la base d'un gradient de bruit du système d'imagerie par résonance magnétique (38).

8. Système d'imagerie par résonance magnétique (38) selon la revendication 7, équipé d'un lit de patient (32), dans lequel
l'interface (1) est formée d'un seul tenant avec le lit du patient (32), et
le lit du patient (32) est un sous-module (3) et peut être connecté à des dispositifs périphériques (6) de types correspondants.

9. Système d'imagerie par résonance magnétique (38) selon la revendication 8, lorsqu'elle dépend de la revendication 5, dans lequel le lit de patient (32) peut être connecté aux dispositifs périphériques de premier type (7) et aux dispositifs périphériques de second type (8).

10. Système d'imagerie par résonance magnétique (38) selon la revendication 8 ou 9 doté d'un dispositif de commande (44), le dispositif de commande (44) étant interconnecté entre le lit de patient (32) et le module hôte (2), dans lequel le lit de patient (32) est un dispositif périphérique (6) et le dispositif de commande (44) correspond au sous-module correspondant (3), de sorte que le lit de patient (32) puisse être commandé avec le module hôte (2).

11. Procédé de fonctionnement d'un système d'imagerie médicale (38) selon l'une quelconque des revendications 7 à 10, comprenant :
la connexion (S1) des dispositifs périphériques (6) à l'interface (1),
l'identification (S2) des dispositifs périphériques compatibles (28) ainsi que des dispositifs périphériques incompatibles (30),
le déclenchement (S3) du verrouillage de sécurité dans le système d'imagerie médicale (38) lorsque le dispositif périphérique incompatible (30) est identifié,
l'identification (S4) des types des dispositifs périphériques compatibles (28),
la présélection (S5) d'une séquence d'imagerie avec l'interface (1) lors de l'identification des dispositifs périphériques compatibles (28),
l'établissement (S6) de l'autre connexion de données de l'interface (1) au système d'imagerie médicale (38), et la réalisation (S7) de la séquence d'imagerie avec le système d'imagerie médicale (38) si aucun verrouillage de sécurité n'a eu lieu.

12. Procédé de fonctionnement d'un système d'imagerie médicale (38) selon l'une quelconque des revendications 7 à 10, comprenant :
la connexion (S1) des dispositifs périphériques (6) au lit de patient (32),
l'identification (S2) des dispositifs périphériques (6) connectés au lit de patient (32),
le déclenchement (S3) du verrouillage de sécurité dans le système d'imagerie médicale (38) lorsque le dispositif périphérique incompatible (30) est identifié,
l'identification (S4) des types des dispositifs périphériques compatibles (28),
la présélection (S5) d'une séquence d'imagerie avec le lit de patient (32) lors de l'identification des dispositifs périphériques compatibles (28),
l'établissement (S6) de l'autre connexion de données du lit de patient (32) au système d'imagerie médicale (38), et
la réalisation (S7) de la séquence d'imagerie avec le système d'imagerie médicale (38) si aucun verrouillage de sécurité n'a eu lieu.

13. Procédé selon la revendication 11 ou 12, comprenant en outre :
la transmission (S7a) de la séquence d'imagerie à l'interface (1), et dans lequel lors de l'étape S7 l'étape supplémentaire suivante est exécutée :
la commande (S8) de paramètres environnementaux avec l'interface (1).

14. Support lisible par ordinateur comprenant des instructions qui, lorsqu'elles sont exécutées par une interface (1) selon l'une quelconque des revendications 1 à 6 dans un système d'imagerie médicale (38), amènent l'interface (1) à réaliser un procédé selon l'une quelconque des revendications 11 à 13.
